(19) Europäisches Patentamt
European Patent Office
Office européen des brevets

(11) Veröffentlichungsnummer: **0 181 471**
**A1**

(12) **EUROPÄISCHE PATENTANMELDUNG**

(21) Anmeldenummer: **85112138.4**

(22) Anmeldetag: **25.09.85**

(51) Int. Cl.[4]: **C 07 D 231/52**
A 61 K 31/415

(30) Priorität: **17.10.84 DE 3438027**

(43) Veröffentlichungstag der Anmeldung:
**21.05.86 Patentblatt 86/21**

(84) Benannte Vertragsstaaten:
**AT BE CH DE FR GB IT LI LU NL SE**

(71) Anmelder: **LUDWIG HEUMANN & CO GMBH**
**Heideloffstrasse 18-28 Postfach 2260**
**D-8500 Nürnberg(DE)**

(72) Erfinder: **Herter, Rolf, Dr. Dipl.-Chem.**
**Bayernstrasse 42**
**D-8540 Schwabach(DE)**

(72) Erfinder: **Schickaneder, Helmut, Dr. Dipl.-chem.**
**Moosäcker 25**
**D-8501 Eckental(DE)**

(72) Erfinder: **Mörsdorf, Peter, Dr. Dipl.-Chem.**
**Untere Bahnhofstrasse 16**
**D-8501 Cadolzburg(DE)**

(72) Erfinder: **Postius, Stefan, Dr.**
**Nunnenbeckstrasse 24**
**D-8500 Nürnberg(DE)**

(72) Erfinder: **Szelenyi, Istvan, Dr.**
**Haendelstrasse 32**
**D-8501 Schwaig(DE)**

(72) Erfinder: **Ahrens, Kurt Henning, Dr.**
**Praterstrasse 9**
**D-8500 Nürnberg(DE)**

(74) Vertreter: **Kraus, Walter, Dr. et al,**
**Patentanwälte Kraus, Weisert & Partner**
**Thomas-Wimmer-Ring 15**
**D-8000 München 22(DE)**

(54) **Pyrazolderivate, Verfahren zu ihrer Herstellung und diese Verbindungen enthaltende Arzneimittel.**

(57) Es werden Pyrazolderivate der allgemeinen Formel I

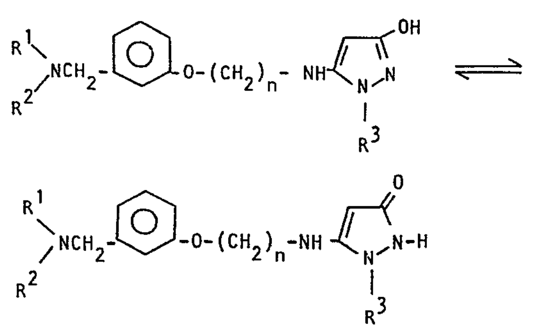

(I)

in der $R^1$ und $R^2$ unabhängig voneinander ein Wasserstoffatom, eine lineare oder verzweigt $C_1$-$C_3$-Alkylgruppe bedeuten oder $R^1$ und $R^2$ zusammen mit dem Stickstoffatom einen 4- bis 7gliedrigen alicyclischen stickstoffhaltigen Heterocyclus bilden, n für eine ganze Zahl von 2 bis 6 steht, $R^3$ ein Wasserstoffatom oder eine Methyl- oder Ethylgruppe bedeutet, sowie die physiologisch annehmbaren Salze und Hydrate davon beschrieben, welche eine hohe selektive Wirkung auf Histamin-$H_2$-Rezeptoren haben. Diese Verbindungen weisen gegenüber anerkannt guten Histamin-$H_2$-Rezeptoren verbesserte pharmakologische Eigenschaften auf. Es wird weiterhin ein Herstellungsverfahren für diese Verbindungen beschrieben.

EP 0 181 471 A1

# Pyrazolderivate, Verfahren zu ihrer Herstellung und diese Verbindungen enthaltende Arzneimittel

## BESCHREIBUNG

Die Erfindung betrifft neue Pyrazolderivate mit einer hohen selektiven Wirkung auf Histamin-$H_2$-Rezeptoren, Verfahren zu ihrer Herstellung und Arzneimittel, die diese Verbindungen enthalten, sowie schließlich die Verwendung dieser Verbindungen in der Therapie.

Als Anti-Ulcusmittel haben Cimetidin und Ranitidin bereits Eingang in die Therapie gefunden. Die Halbwertszeiten von Cimetidin und Ranitidin sind jedoch verhältnismäßig kurz. Dadurch wird es nötig, mehrfach täglich Tabletten mit Dosiseinheiten von 160 - 300 mg in einer therapeutisch festgelegten Form zu verabreichen. Es besteht weiterhin ein Bedarf an Anti-Ulcusmitteln, die in ihrem pharmakologischen Wirkprofil dem Cimetidin und Ranitidin überlegen sind. Die erfindungsgemäßen Verbindungen zeigen aufgrund ihrer spezifischen $H_2$-antagonistischen Aktivität eine Hemmung der Magensäuresekretion, wenn diese durch Histaminagonisten stimuliert wird. Ash und Shild, "Brit. J. Pharmacol. Chemother.", 27, 427 (1966) und Black et al., "Nature", 236, 385 (1972).

Die pharmakologische Aktivität dieser Verbindungen kann nach einer modifizierten Methode gemäß der DE OS 2734070 beim perfundierten Rattenmagen gezeigt werden oder durch die Ermittlung der $pA_2$-Werte in vitro am Meerschweinchenvorhof (vergleiche Ariens, Molec. Pharmac., Band 1, Academic Press, New York, 1964) nachgewiesen werden.

Weiterhin kann die $H_2$-antagonistische Wirkung an wachen Heidenhain-Pouch-Hunden nach der Methode von Black et al., "Nature" 236, 385 (1972) und an wachen Fistelkatzen gezeigt werden.

Die neuen Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden. Nachdem Hemmstoffe für Histamin-$H_2$-Rezeptoren sowohl bezüglich der basalen Magensäuresekretion als auch der durch Gastrin, Histamin, Metacholin oder Nahrung induzierten Magensäuresekretion Hemmwirkung besitzen, können sie für die Behandlung von peptischen Ulcera, die durch übermäßige Sekretion von Magensäure verursacht sind, und bei der Therapie hyperacider Gastritis verwendet werden.

Der Erfindung liegt die Aufgabe zugrunde, neue Hemmstoffe für Histamin-$H_2$-Rezeptoren mit verbesserter Wirksamkeit zur Verfügung zu stellen. Diese Aufgabe wird durch die Erfindung gelöst.

Gegenstand der Erfindung sind daher neue Pyrazolderivate der allgemeinen Formel I

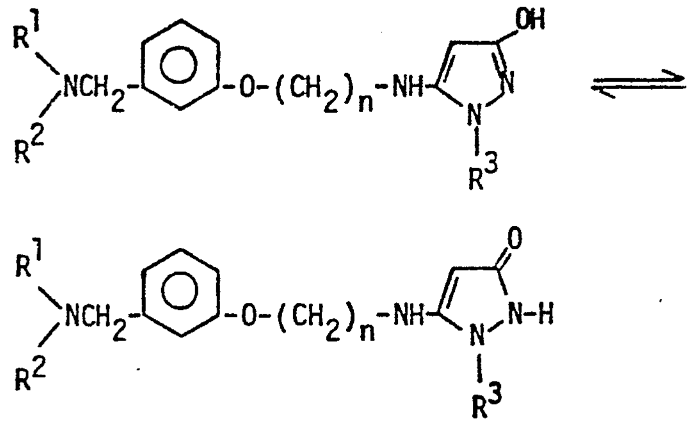

( I )

in der $R^1$ und $R^2$ unabhängig voneinander ein Wasserstoffatom, eine lineare oder verzweigte $C_1$-$C_3$-Alkylgruppe bedeuten oder $R^1$ und $R^2$ zusammen mit dem Stickstoffatom einen 4- bis 7gliedrigen alicyclischen stickstoffhaltigen Heterocyclus bilden, n für eine ganze Zahl von 2 bis 6 steht, $R^3$ ein Wasserstoffatom oder eine Methyl- oder Ethylgruppe bedeutet, sowie die physiologisch annehmbaren Salze und Hydrate davon.

In der allgemeinen Formel I bedeuten $R^1$ und $R^2$ unabhängig voneinander ein Wasserstoffatom oder eine lineare oder verzweigtkettige $C_1$-$C_3$-Alkylgruppe. Beispiele für solche linearen oder verzweigtkettigen Alkylgruppen sind die Methyl-, Ethyl-, i- und n-Propylgruppe, wobei die Methyl- und die Ethylgruppe bevorzugt werden. Die Substituenten $R^1$ und $R^2$ können aber auch zusammen mit dem Stickstoffatom, an das sie gebunden sind, einen stickstoffhaltigen heterocyclischen Ring mit 4 bis 7 Gliedern bilden. Beispiele für diese Ringe sind der Azetidin-, der Pyrrolidin-, der Piperidin- und der Homopiperidinring, wobei der Pyrrolidin- und Piperidinring bevorzugt werden. n ist eine ganze Zahl von 2 bis 6, vorzugsweise 3. $R^3$ steht für ein Wasserstoffatom oder eine

Methyl- oder Ethylgruppe, vorzugsweise eine Methylgruppe.

Eine bevorzugte Gruppe von erfindungsgemäßen Verbindungen ist dadurch gekennzeichnet, daß in der allgemeinen Formel I $R^1$ für ein Wasserstoffatom steht und $R^2$ ebenfalls ein Wasserstoffatom oder eine lineare oder verzweigte $C_1$-$C_3$-Alkylgruppe, wie zum Beispiel eine Methyl-, Ethyl-, Propyl- oder Isopropylgruppe, bedeutet.

Eine weitere bevorzugte Gruppe von erfindungsgemäßen Verbindungen ist dadurch gekennzeichnet, daß $R^1$ für eine lineare oder verzweigte $C_1$-$C_3$-Alkylgruppe, wie zum Beispiel eine Methyl-, Ethyl- oder Propylgruppe, insbesondere eine Methylgruppe, steht und $R^2$ ebenfalls für eine lineare oder verzweigte $C_1$-$C_3$-Alkylgruppe, vorzugsweise eine Methylgruppe, steht.

Eine andere bevorzugte Gruppe von erfindungsgemäßen Verbindungen ist dadurch gekennzeichnet, daß $R^1$ und $R^2$ für Methyl stehen.

Beispiele für bevorzugte Verbindungen sind die folgenden Verbindungen:
1-Methyl-5-[3-[3-(piperidinomethyl)phenoxy]propylamino]-3-hydroxy-1H-pyrazol,
1-Methyl-5-[3-[3-(pyrrolidinomethyl)phenoxy]propylamino]-3-hydroxy-1H-pyrazol,
1-Methyl-5-[3-[3-(dimethylaminomethyl)phenoxy]propylamino]-3-hydroxy-1H-pyrazol,
1-Methyl-5-[4-[3-(piperidinomethyl)phenoxy]butylamino]-3-hydroxy-1H-pyrazol,
1-Methyl-5-[4-[3-(pyrrolidinomethyl)phenoxy]butylamino]-3-hydroxy-1H-pyrazol,
1-Methyl-5-[6-[3-(piperidinomethyl)phenoxy]hexylamino]-3-hydroxy-1H-pyrazol und
5-[3-[3-(piperidinomethyl)phenoxy]propylamino]-3-hydroxy-1H-pyrazol.

Die erfindungsgemäßen Verbindungen können in verschiedenen tautomeren Formen gemäß dem obigen Formelpaar vorliegen. Die Erfindung soll daher auch sämtliche tautomeren Verbindungen sowie die physiologisch annehmbaren Hydrate und Salze davon umfassen. Die Salze werden mit anorganischen und organischen Säuren in an sich bekannter Weise hergestellt. So können diese Salze zum Beispiel mit Mineralsäuren, wie Chlor-, Brom-, Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure etc., gebildet werden.

Die erfindungsgemäßen Verbindungen können durch ein Verfahren hergestellt werden, das dadurch gekennzeichnet ist, daß man eine Verbindung der allgemeinen Formel II

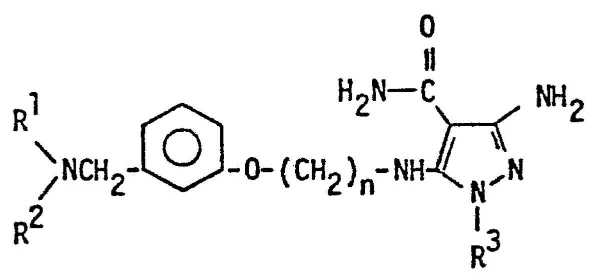

(II)

in der $R^1$, $R^2$, $R^3$ und n die oben angegebenen Bedeutungen haben, in saurer wäßriger Lösung zu einer erfindungsgemäßen Verbindung der allgemeinen Formel I umsetzt und die erhaltene Verbindung gegebenenfalls in ihr physiologisch annehmbares Salz umwandelt.

Die Umsetzung wird beispielsweise in konzentrierter, wäßriger salzsaurer Lösung bei Rückflußtemperatur durchgeführt. Die Reaktionszeit beträgt mehrere Stunden, zum Beispiel 18 Stunden. Die Aufarbeitung und Isolierung der angestrebten Verbindung erfolgt auf übliche Weise, beispielsweise durch chromatographische Reinigung und Kristallisation.

Die Herstellung der im folgenden beschriebenen Zwischenprodukte läßt sich analog R. Gompper und W. Töpfl (Chem. Ber. 95, 2871 und Chem. Ger. 95, 2881 (1962)) durchführen.

Die erfindungsgemäßen Verbindungen können zur Verabreichung in jeder beliebigen Weise formuliert werden. Die Erfindung umfaßt daher auch

Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutisch annehmbaren Trägern oder Verdünnungsmitteln hergestellt werden.

Die erfindungsgemäßen Verbindungen können daher für die orale, bukkale, topische, parenterale oder rektale Verabreichung formuliert werden, wobei die orale Verabreichung bevorzugt wird.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/ oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert

werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die erfindungsgemäßen Verbindungen zeichnen sich gegenüber anerkannt guten Arzneimitteln der gleichen Wirkungsrichtung durch eine Verbesserung des pharmakologischen Wirkprofils aus. Dies ergibt sich aus den Ergebnissen der im folgenden dargestellten pharmakologischen Vergleichsuntersuchungen.

Pharmakologische Untersuchungen:

Versuchsmodell: wache Fistelkatze
(Stimulator: Histamin)
CIMETIDIN (Vergleich) i.V. $ID_{50}$ 1,4 µmol/kg
Beispiel 1 i.V. $ID_{50}$ 0,03 µmol/kg

Beispiel 1

a) Herstellung von

2-Cyano-3-methylthio-3-[3-[3-(piperidinomethyl) phenoxy]propylamino]-propennitril

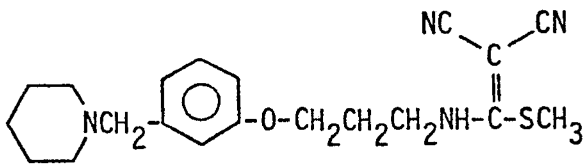

12.4 g (73 mmol) Bis-methylthio-methylenmalondinitril in 200 ml Diethylether werden bei Raumtemperatur mit 18.1 g (73 mmol) 3-[3-(Piperidinomethyl)phenoxy]propanamin versetzt. Man rührt noch 1 Stunde bei Raumtemperatur und 1 Stunde unter Rückfluß und entfernt dann das Lösungsmittel im Vakuum. Das verbleibende hellrote, zähe Öl wird ohne Reinigung weiter umgesetzt.

Ausbeute: 25.7 g (95 % d.Th.)

Rf: 0.45 ($Al_2O_3$ neutral; Ethylacetat/Petrolether 5/2 )

$C_{20}H_{26}N_4OS$ (370)

$^1$H-NMR-Daten:
($CDCl_3$, TMS als interner Standard)

$\delta$ = 1.50 (m) 6 H,
2.15 (m) 2 H,
2.39 (m) 4 H,
2.56 (s) 3 H,
3.43 (s) 2 H,
3.83 (t) 2 H,
4.12 (t) 2 H,
6.5 - 7.4 (m) 5 H (1 H austauschbar mit $D_2O$) ppm.

b) Herstellung von

4-Cyano-1-methyl-$N^5$-[3-[3-(piperidinomethyl)phenoxy]propyl]-pyrazol-3,5-diamin

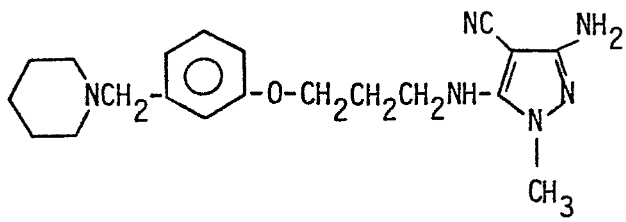

25.7 g (69 mmol) 2-Cyano-3-methylthio-3-[3-[3-(piperidinomethyl)phenoxy]propylamino]-propennitril werden in 30 ml Ethanol gelöst und bei 85 °C zu 40 ml Methylhydrazin in 80 ml Wasser gegeben. Man rührt noch 1 Stunde bei dieser Temperatur, läßt abkühlen, verdünnt mit etwas Wasser und extrahiert mit Essigsäureethylester. Nach dem Trocknen über Natriumsulfat wird die organische Phase eingeengt und das verbleibende Öl aus Ethylacetat/Diethylether kristallisiert.

Farblose Kristalle vom Schmelzpunkt 102 - 104 °C

Ausbeute: 16.8 g (66 % d.Th.)

Rf: 0.46 ($Al_2O_3$ neutral; Ethylacetat/Isopropanol 95/5)

$C_{20}H_{28}N_6O$ (368)

| | |
|---|---|
| $^1$H-NMR-Daten: | $\delta$ = 1.42 (m) 6 H, |
| ($d_6$-DMSO, TMS als interner | 2.00 (m) 2 H, |
| Standard) | 2.30 (m) 4 H, |
| | 3.30 (s) 3 H, |
| | 3.37 (s) 2 H, |
| | 3.2 - 3.65 (m) 2 H, |
| | 4.04 (t) 2 H, |
| | 4.98 (s) 2 H,(austauschbar mit $D_2O$) |
| | 6.42 (t) 1 H (austauschbar mit $D_2O$) |
| | 6.7 - 7.4 (m) 4 H ppm. |

c) Herstellung von

4-Carboxamido-1-methyl-$N^5$[3-[3-(piperidinomethyl) phenoxy] propyl] - pyrazol-3,5-diamin

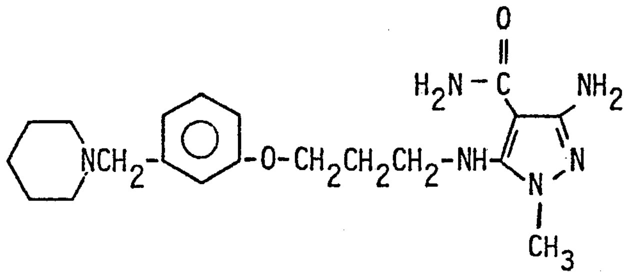

5.9 g (16.5 mmol) 4-Cyano-1-methyl-$N^5$-[3-[3-(piperidinomethyl)phenoxy] propyl]-pyrazol-3,5-diamin werden in 100 ml 50 %-igem Ethanol mit 6.6 g Natriumhydroxid 16 Stunden unter Rückfluß gekocht. Man verdünnt mit Wasser, extrahiert mit Ethylacetat und trocknet über Natriumsulfat. Nach dem Einengen im Vakuum verbleibt ein ockerfarbener Feststoff, der aus Ethylacetat umkristallisiert wird.

Farblose Kristalle vom Schmelzpunkt 100 - 102 °C

Ausbeute: 3.8 g (60 % d.Th.)

Rf: 0.35 (Kieselgel; Ethylacetat/Methanol/TAA 85/10/5 )

$C_{20}H_{30}N_6O_2$ (386)

| | |
|---|---|
| $^1$H-NMR-Daten: | $\delta$ = 1.50 (m) 6 H, |
| ($CDCl_3$, TMS als | 2.03 (m) 2 H, |
| interner Standard) | 2.37 (m) 4 H, |
| | 3.25 - 3.6 (m) 2 H, |
| | 3.43 (s) 2 H, |
| | 3.61 (s) 3 H, |
| | 3.95 (s) 2 H (austauschbar mit $D_2O$) |
| | 4.08 (t) 2 H, |
| | 5.80 (t) 1 H (austauschbar mit $D_2O$) |
| | 6.19 (s) 2 H (austauschbar mit $D_2O$) |
| | 6.7 - 7.4 (m) 4 H ppm. |

d)

1-Methyl-5-[3-[3-(piperidinomethyl)phenoxy]propylamin]-3-hydroxy-1H-pyrazol
bzw.
5-Amino-1-methyl-$N^5$-[3-[3-(piperidinomethyl) phenoxy]propyl]-1,2-dihydro-pyrazol-3-on

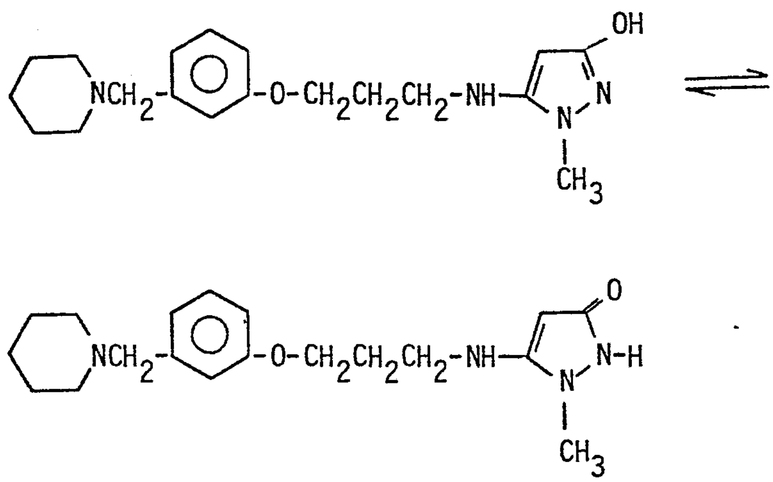

3.8 g (9.8 mmol) 4-Carboxamido-1-methyl-$N^5$-[3-[3-(piperidinomethyl) phenoxy]propyl]-pyrazol-3,5-diamin werden in 150 ml 20 %-iger wäßriger Salzsäure 24 Stunden unter Rückfluß gekocht. Das Reaktionsgemisch wird im Vakuum eingeengt, in Wasser gelöst, mit Kaliumcarbonat versetzt und mit Ethylacetat extrahiert. Nach dem Trocknen über Natriumsulfat und Einengen des Lösungsmittels im Vakuum verbleibt ein brauner Feststoff, der aus Ethylacetat umkristallisiert wird.

Farblose Kristalle vom Schmelzpunkt 128.5 - 129.5 °C

Ausbeute: 1.8 g (53 % d.Th.)

Rf: 0.60 (Kieselgel; Methylenchlorid / Methanol / TAA 85/10/5 )

| | | | |
|---|---|---|---|
| $C_{19}H_{28}N_4O_2$ (344) | Ber.: | C 66,25 | H 8,19 |
| | Gef.: | C 65,91 | H 8,05 |

| | |
|---|---|
| $^1$H-NMR-Daten: (d$_6$-DMSO, TMS als interner Standard) | $\delta$ = 1.44 (m) 6 H,<br>1.95 (m) 2 H,<br>2.32 (m) 4 H,<br>3.08 (m) 2 H,<br>3.19 (s) 3 H,<br>3.40 (s) 2 H,<br>4.05 (t) 2 H,<br>4.51 (s) 1 H,(austauschbar mit $D_2O$)<br>5.84 (t) 1 H (austauschbar mit $D_2O$)<br>6.7 - 7.4 (m) 4 H,<br>8.4 - 10.0 (breit) 1 H (austauschbar mit $D_2O$) ppm. |

Beispiel 2

a) 2-Cyano-3-methylthio-3-[3-[3-(pyrrolidinomethyl)phenoxy]propylamino]propennitril

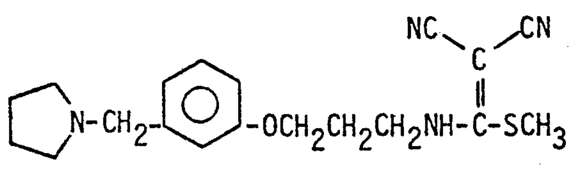

Die Herstellung erfolgt analog Beispiel 1 a.

Rf: 0.35 ($Al_2O_3$ neutral; Ethylacetat / Petrolether 5/2)

$C_{19}H_{24}N_4OS$ (356)

| | |
|---|---|
| $^1$H-NMR-Daten: (CDCl$_3$, TMS als interner Standard) | δ = 1.80 (m) 4 H,<br>2.19 (m) 2 H,<br>2.58 (m) 4 H,<br>2.62 (s) 3 H,<br>3.64 (s) 2 H,<br>3.84 (m) 2 H,<br>4.15 (t) 2 H,<br>6.6 - 7.4 (m) 5 H (1 H austauschbar mit $D_2O$) ppm. |

b) 4-Cyano-1-methyl-N$^5$-[3-[3-(pyrrolidinomethyl)phenoxy]propyl]-pyrazol-3,5-diamin

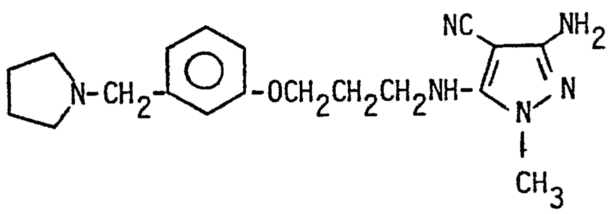

Die Herstellung erfolgt analog Beispiel 1 b.

Farblose Kristalle vom Schmelzpunkt 102 - 104 °C

Rf: 0.4 ($Al_2O_3$ neutral; Ethylacetat / Isopropanol 95/5)

$C_{19}H_{26}N_6O$ (354)

| | |
|---|---|
| $^1$H-NMR-Daten: (CDCl$_3$, TMS als interner Standard) | δ = 1.78 (m) 4 H,<br>2.15 (m) 2 H,<br>2.52 (m) 4 H,<br>3.38 (s) 3 H,<br>3.60 (s) 2 H,<br>3.72 (t) 2 H,<br>3.97 (s) 2 H (austauschbar mit $D_2O$) |

4.65 (t) 1 H (austauschbar mit $D_2O$)
6.8 - 7.4 (m) 4 H ppm.

c) 4-Carboxamido-1-methyl-$N^5$-[3-[3-(pyrrolidinomethyl)phenoxy] propyl] -pyrazol-3,5-diamin

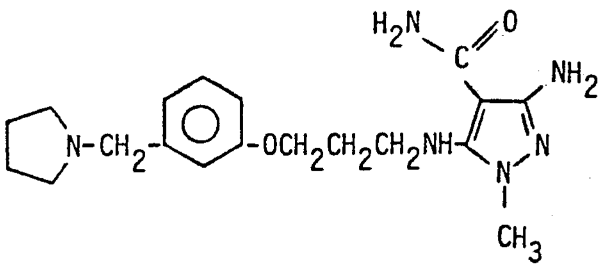

Die Herstellung erfolgt analog Beispiel 1 c.

Farblose Kristalle vom Schmelzpunkt 78 - 80 °C

Rf: 0.24 (Kieselgel; Ethylacetat / Methanol / TAA 85/10/5 )

$C_{19}H_{28}N_6O_2$ (372)

| | |
|---|---|
| $^1$H-NMR-Daten: (CDCl$_3$, TMS als interner Standard) | $\delta$ = 1.75 (m) 4 H, |
| | 2.01 (m) 2 H, |
| | 2.50 (m) 4 H, |
| | 3.35 (m) 2 H, |
| | 3.61 (s) 5 H, |
| | 3.90 (s) 2 H (austauschbar mit $D_2O$), |
| | 4.07 (t) 2 H, |
| | 5.71 (t) 1 H (austauschbar mit $D_2O$), |
| | 6.10 (s) 2 H (austauschbar mit $D_2O$), |
| | 6.7 - 7.4 (m) 4 H ppm. |

d) 1-Methyl-5-[3-[3-(pyrrolidinomethyl) phenoxy] propylamino] -3-hydroxy-1H-pyrazol

bzw.

5-Amino-1-methyl-$N^5$-[3-[3-( pyrrolidinomethyl)phenoxy] propyl] -1,2-dihydro-pyrazol-3-on

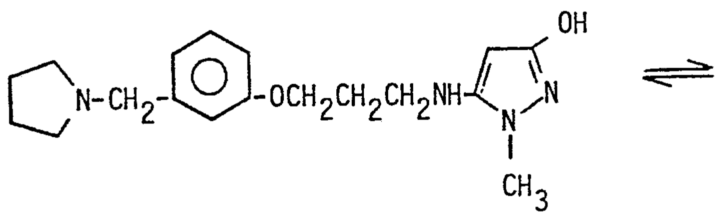

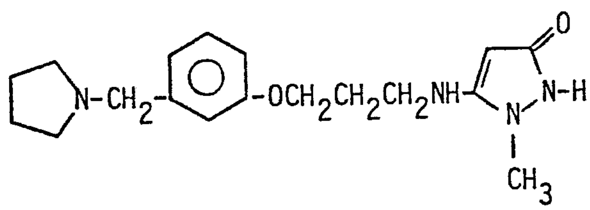

Die Herstellung erfolgt analog Beispiel 1 d.

Farblose Kristalle vom Schmelzpunkt 120 - 123 °C

Rf: 0.23 (Kieselgel; Methylenchlorid / Methanol / TÄA 100/10/5 )

$C_{18}H_{26}N_4O_2$ (330)

$^1$H-NMR-Daten: (d$_6$-DMSO, TMS als interner Standard)

$\delta$ = 1.68 (m) 4 H,
1.96 (m) 2 H,
2.43 (m) 4 H,
3.07 (m) 2 H,
3.15 (s) 3 H,
3.56 (s) 2 H,
4.04 (t) 2 H,
4.50 (s) 1 H (austauschbar mit $D_2O$),
5.86 (t) 1 H (austauschbar mit $D_2O$),
6.8 - 7.4 (m) 4 H,
6.5 - 9.0 (breit) 1 H (austauschbar mit $D_2O$) ppm.

Beispiel 3

a) 2-Cyano-3-methylthio-3-[6-[3-(piperidinomethyl)phenoxy]hexylamino] propennitril

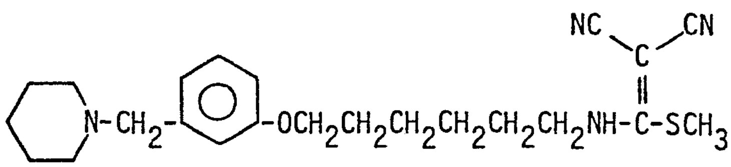

Die Herstellung erfolgt analog Beispiel 1 a.

Rf: 0.5 ($Al_2O_3$ neutral; Ethylacetat / Petrolether 5/2)

$C_{23}H_{32}N_4OS$ (412)

$^1$H-NMR-Daten: (CDCl$_3$, TMS als interner Standard)

$\delta$ = 1.2 - 2.0 (m) 14 H,
2.36 (m) 4 H,
2.64 (s) 3 H,
3.42 (s) 2 H,
3.54 (t) 2 H,
3.95 (t) 2 H,
5.5 - 6.3 (breit) 1 H (austauschbar mit $D_2O$)
6.6 - 7.4 (m) 4 H ppm.

b) 4-Cyano-1-methyl-N$^5$-[6-[3-(piperidinomethyl)phenoxy]hexyl]-pyrazol-3,5-diamin

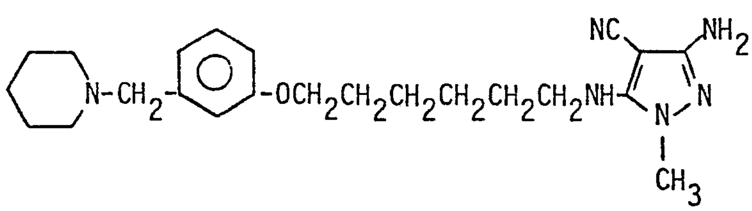

Die Herstellung erfolgt analog Beispiel 1 b.

Farblose Kristalle vom Schmelzpunkt 47 - 47.5 °C

Rf: 0.55 ($Al_2O_3$ neutral; Ethylacetat / Isopropanol 95/5 )

$C_{23}H_{34}N_6O$ (410)

| | |
|---|---|
| $^1$H-NMR-Daten: | $\delta$ = 1.25 - 2.1 (m) 14 H, |
| ($CDCl_3$, TMS als | 2.37 (m) 4 H, |
| interner Standard) | 3.38 (s) 3 H, |
| | 3.44 (s) 2 H, |
| | 3.50 (m) 2 H, |
| | 3.7 - 4.2 (m) 5 H (3 H austauschbar mit $D_2O$), |
| | 6.7 - 7.4 (m) 4 H ppm. |

c) 4-Carboxamido-1-methyl-$N^5$-[6-[3-(piperidinomethyl) phenoxy]hexyl]-pyrazol-3,5-diamin

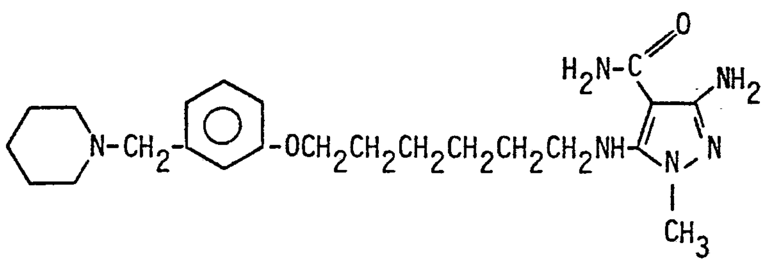

Die Herstellung erfolgt analog Beispiel 1 c

Farblose Kristalle vom Schmelzpunkt 54 - 56 °C

Rf: 0.36 (Kieselgel; Ethylacetat / Methanol / TAA 85/10/5 )

$C_{23}H_{36}N_6O_2$ (428)

| | |
|---|---|
| $^1$H-NMR-Daten: | $\delta$ = 1.2 - 2.1 (m) 14 H, |
| ($CDCl_3$, TMS als | 2.43 (m) 4 H, |
| interner Standard) | 3.23 (m) 2 H, |
| | 3.50 (s) 2 H, |
| | 3.65 (s) 3 H, |

4.01 (t) 2 H,
4.10 (s) 2 H (austauschbar mit $D_2O$),
5.83 (t) 1 H (austauschbar mit $D_2O$),
6.35 (s) 2 H (austauschbar mit $D_2O$),
6.8 - 7.5 (m) 4 H ppm.

d) 1-Methyl-5-[6-[3-(piperidinomethyl)phenoxy]hexylamino]-3-hydroxy-1H-pyrazol

bzw.

5-Amino-1-methyl-$N^5$-[6-[3-(piperidinomethyl)phenoxy]hexyl]-1,2-dihydro-pyrazol-3-on

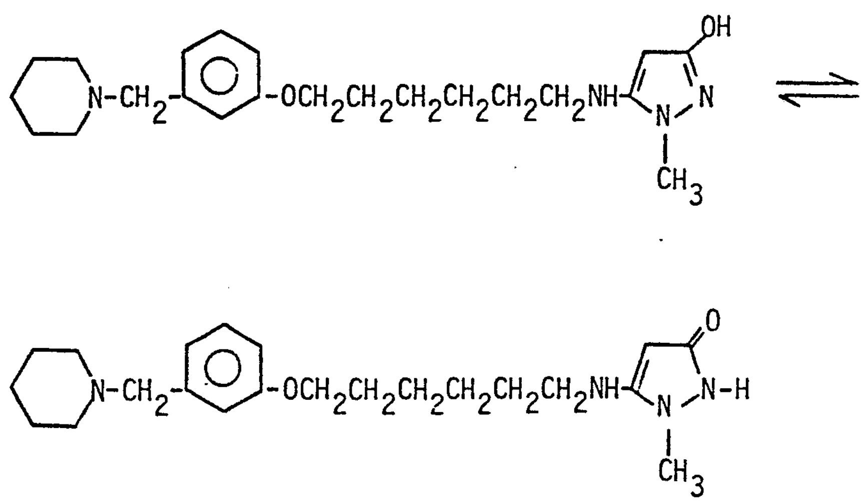

Die Herstellung erfolgt analog Beispiel 1 d.

Farblose Kristalle vom Schmelzpunkt 106 - 109 °C

Rf: 0.21 (Kieselgel; Ethylacetat / Methanol / TÄA 85/10/5 )

$C_{22}H_{34}N_4O_2$ (386)

$^1$H-NMR-Daten:
($d_6$-DMSO, TMS als interner Standard)

$\delta$ = 1.2 - 2.0 (m) 14 H,
2.30 (m) 4 H,
2.92 (m) 2 H,
3.09 (s) 3 H,
3.0 - 4.0 (breit) 1 H (austauschbar mit $D_2O$),
3.37 (s) 2 H,
3.94 (t) 2 H,
4.42 (s) 1 H (austauschbar mit $D_2O$)
5.78 (t) 1 H (austauschbar mit $D_2O$)
6.7 - 7.4 (m) 4 H ppm.

Beispiel 4

1-Methyl-5-[4-[3-(piperidinomethyl)phenoxy]butylamino]-3-hydroxy-1H-pyrazol
bzw.
5-Amino-1-methyl-$N^5$-[4-[3-(piperidinomethyl)phenoxy]butyl]-1,2-dihydro-pyrazol-3-on

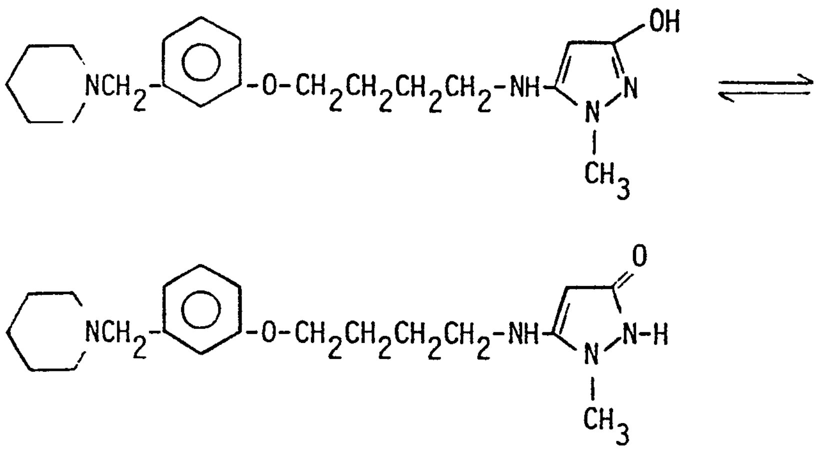

Die Herstellung erfolgt analog den Beispielen 1 a - d.

Farblose Kristalle vom Schmelzpunkt 140° C

Rf: 0.35 (Kieselgel; Chloroform /Methanol/Ammoniak 85/13/2)

$C_{20}H_{30}N_4O_2$ (358)

| | |
|---|---|
| $^1$H-NMR-Daten: | $\delta$ = 1.41 (m) 6 H, |
| ($d_6$-DMSO, TMS als | 1.73 (m) 4 H, |
| interner Standard) | 2.30 (m) 4 H, |
| | 3.00 (m) 2 H, |
| | 3.12 (s) 3 H, |
| | 3.37 (s) 2 H, |
| | 3.97 (m) 2 H, |
| | 4.46 (s) 1 H (austauschbar mit $D_2O$), |
| | 5.81 (t) 1 H (austauschbar mit $D_2O$), |

6.7 - 7.4 (m) 4 H,
8.7 - 9.5 (breit) 1 H (austauschbar mit $D_2O$) ppm.

## Beispiel 5

1-Methyl-5-[4-[3-(pyrrolidinomethyl)phenoxy]butylamino]-3-hydroxy-1H-pyrazol
bzw.
5-Amino-1-methyl-$N^5$-[4-[3-(pyrrolidinomethyl)phenoxy]butyl]-1,2-dihydropyrazol-3-on

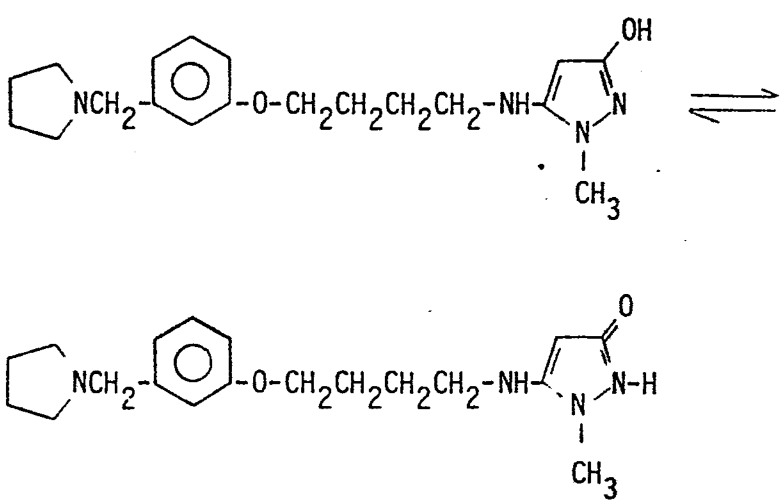

Die Herstellung erfolgt analog den Beispielen 1 a - d.

Farblose Kristalle vom Schmelzpunkt 115 - 116° C

Rf: 0.33 (Kieselgel; Chloroform/Methanol/Ammoniak 85/13/2 )

$C_{19}H_{28}N_4O_2$ (344)

$^1$H-NMR-Daten:
($d_6$-DMSO, TMS als
interner Standard)

$\delta$ = 1.65 (m) 8 H,
2.41 (m) 4 H,
3.0 (m) 2 H,
3.11 (s) 3 H,
3.52 (s) 2 H,
3.97 (m) 2 H,
4.46 (s) 1 H (austauschbar mit $D_2O$),
5.82 (m) 1 H (austauschbar mit $D_2O$),
6.7 - 7.4 (m) 4 H,
8.5 - 9.7 (breit) 1 H (austauschbar mit $D_2O$) ppm.

## Beispiel 6

1-Ethyl-5-[3-[3-(piperidinomethyl)phenoxy]propylamino]-3-hydroxy-1H-pyrazol
bzw.
5-Amino-1-ethyl-$N^5$-[3-[3-(piperidinomethyl)phenoxy]propyl]-1,2-dihydro-pyrazol-3-on

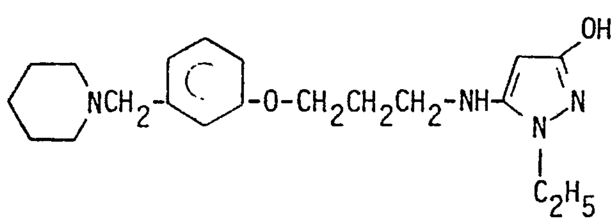

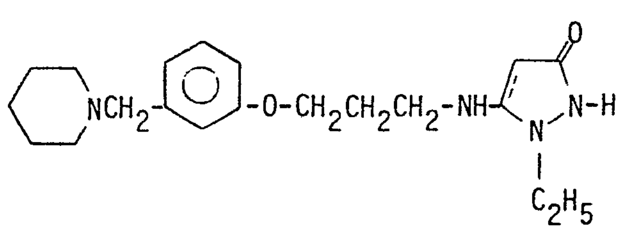

Die Herstellung erfolgt analog den Beispielen 1 a - d mit Ethylhydrazin.

0181471

Farblose Kristalle vom Schmelzpunkt 134 - 135° C

Rf: 0.50 (Kieselgel; Chloroform/Methanol/Ammoniak 85/13/2)

$C_{20}H_{30}N_4O_2$ (358)

| | |
|---|---|
| $^1$H-NMR-Daten: (d$_6$-DMSO, TMS als interner Standard) | $\delta$ = 1.06 (t) 3 H,<br>1.43 (m) 6 H,<br>1.96 (m) 2 H,<br>2.32 (m) 4 H,<br>3.12 (m) 2 H,<br>3.40 (s) 2 H,<br>3.50 (q) 2 H,<br>4.03 (t) 2 H,<br>4.49 (s) 1 H (austauschbar mit $D_2O$),<br>5.93 (t) 1 H (austauschbar mit $D_2O$),<br>6.7 - 7.4 (m) 4 H,<br>8.5 - 9.5 (breit) 1 H (austauschbar mit $D_2O$) ppm. |

PATENTANSPRÜCHE

1. Pyrazolderivate der allgemeinen Formel I

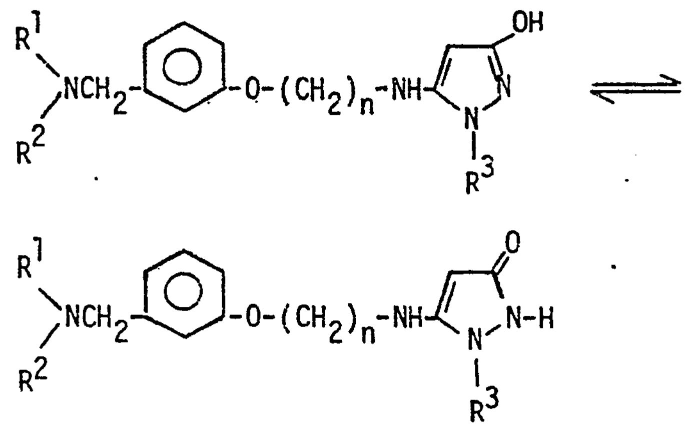

( I )

in der $R^1$ und $R^2$ unabhängig voneinander ein Wasserstoffatom, eine lineare oder verzweigte $C_1$-$C_3$-Alkylgruppe bedeuten oder $R^1$ und $R^2$ zu-

sammen mit dem Stickstoffatom einen 4- bis 7gliedrigen alicyclischen stickstoffhaltigen Heterocyclus bilden, n für eine ganze Zahl von 2 bis 6 steht, $R^3$ ein Wasserstoffatom oder eine Methyl- oder Ethylgruppe bedeutet, sowie die physiologisch annehmbaren Salze und Hydrate davon.

2. Pyrazolderivate nach Anspruch 1, dadurch gekennzeichnet, daß $R^1$ und $R^2$ zusammen mit dem Stickstoffatom einen 5- bis 6-gliedrigen alicyclischen heterocyclischen Ring bilden, n die Zahl 3 bedeutet und $R^3$ für Methyl steht, sowie die physiologisch annehmbaren Salze und Hydrate davon.

3. 1-Methyl-5-[3-[3-(piperidinomethyl)phenoxy]propylamino]-3-hydroxy-1H-pyrazol und die physiologisch annehmbaren Salze davon.

4. Verfahren zur Herstellung von Pyrazolverbindungen nach den Ansprüchen 1 bis 3, dadurch gekennzeichnet, daß man eine Verbindung der allgemeinen Formel II

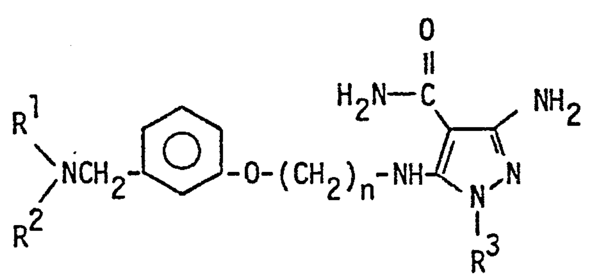

(II)

in der $R^1$, $R^2$, $R^3$ und n die in Anspruch 1 angegebenen Bedeutungen haben, in saurer wäßriger Lösung zu einer erfindungsgemäßen Verbindung der allgemeinen Formel I umsetzt und die erhaltene Verbindung gegebenenfalls in ihr physiologisch annehmbares Salz umwandelt.

5. Arzneimittel, dadurch gekennzeichnet, daß es eine Verbindung nach einem der Ansprüche 1 bis 3 zusammen mit mindestens einem inerten pharmazeutisch annehmbaren Träger oder Verdünnungsmittel enthält.

PATENTANSPRÜCHE

für den Vertragsstaat AT

1. Verfahren zur Herstellung von Pyrazolderivaten der allgemeinen Formel I

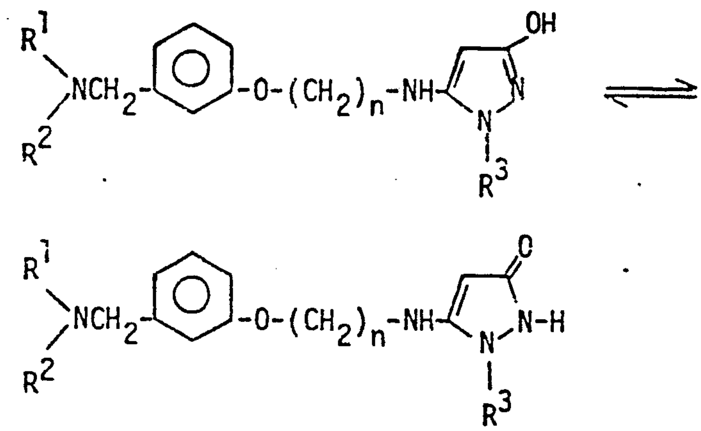

( I )

in der $R^1$ und $R^2$ unabhängig voneinander ein Wasserstoffatom, eine lineare oder verzweigte $C_1$-$C_3$-Alkylgruppe bedeuten oder $R^1$ und $R^2$ zusammen mit dem Stickstoffatom einen 4- bis 7gliedrigen alicyclischen stickstoffhaltigen Heterocyclus bilden, n für eine ganze Zahl von 2 bis 6 steht, $R^3$ ein Wasserstoffatom oder eine Methyl- oder Ethylgruppe bedeutet, sowie der physiologisch annehmbaren Salze und Hydrate davon, dadurch gekennzeichnet, daß man eine Verbindung der allgemeinen Formel II

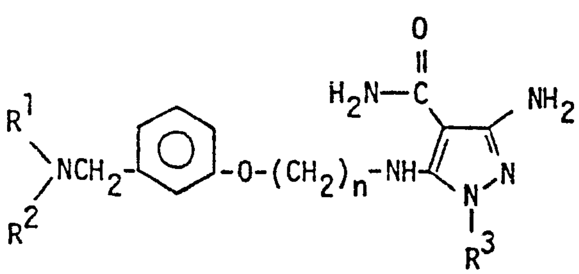

(II)

in der $R^1$, $R^2$, $R^3$ und n die oben angegebenen Bedeutungen haben, in saurer wäßriger Lösung zu einer erfindungsgemäßen Verbindung der allgemeinen Formel I umsetzt und die erhaltene Verbindung gegebenenfalls in ihr physiologisch annehmbares Salz und/oder Hydrat umwandelt.

2. Verfahren nach Anspruch 1 zur Herstellung von Verbindungen, bei denen $R^1$ und $R^2$ zusammen mit dem Stickstoffatom einen 5- bis 6gliedrigen alicyclischen heterocyclischen Ring bilden, n die Zahl 3 bedeutet und $R^3$ für Methyl steht.

3. Verfahren nach Anspruch 1 oder 2 zur Herstellung von 1-Methyl-5-[3-[3-(piperidinomethyl)phenoxy]propylamino]-3-hydroxy-1H-pyrazol und der physiologisch annehmbaren Salze davon.

Europäisches Patentamt

# EUROPÄISCHER RECHERCHENBERICHT

0181471

Nummer der Anmeldung

EP 85 11 2138

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| | Keine Entgegenhaltungen<br>----- | | C 07 D 231/52<br>A 61 K 31/415 |
| | | | RECHERCHIERTE SACHGEBIETE (Int Cl.4) |
| | | | C 07 D 231/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 27-01-1986 | DE BUYSER I.A.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument